# EUROPÄISCHE PATENTSCHRIFT

(11) **EP 2 394 614 B1**
(45) Veröffentlichungstag und Bekanntmachung des Hinweises auf die Patenterteilung: **06.03.2013**
(21) Anmeldenummer: 10165626.2
(22) Anmeldetag: 11.06.2010
(51) Int. Cl.: A61F 5/41

(54) **Befestigungselement für einen Penis**
Fastening element for a penis
Elément de fixation pour un pénis

(43) Veröffentlichungstag der Anmeldung: 14.12.2011
(73) Patentinhaber: Phalomed Holdings LTD., Malta (MT)
(72) Erfinder: Jochum, Herbert, 82541, Münsing (DE)
(74) Vertreter: Köster, Hajo

(56) Entgegenhaltungen:
- DE-B3-102007 020 236
- DE-U1- 9 210 914
- DE-U1-202006 017 667

## Beschreibung

Die Erfindung betrifft ein Befestigungselement für einen Penis zum Verbinden mit einer Penisextensionsvorrichtung, das mit einem Auflageelement sowie einem Fixierelement für den Penis ausgestattet ist.

Penisextensionsgeräte bzw. -Vorrichtungen sind in den verschiedensten Ausgestaltungen bekannt, man vergleiche beispielsweise DE 100 01 331 A1, US 5 707 341 und EP 1 779 822 A1.

Diesen Geräten ist gemeinsam, dass ein länger wirkenden Zug auf den Penis ausgeübt wird, so dass sich neues Gewebe bildet und zu einer Vergrößerung des Penis führt. Mit diesen Vorrichtungen können bestimmte Erkrankungen behandelt werden. Es handelt sich hier somit nicht um Erektionshilfen oder Ähnliches. Vielmehr wird ein Zug auf den Penis im nicht erigierten Zustand ausgeübt.

Dazu ist es erforderlich, die Penisextensionsvorrichtung mit dem Penis zu verbinden, damit dieser Zug ausgeübt werden kann. Selbstverständlicherweise sollte der Ansatzpunkt für die Zugübermittlung am distalen Ende des Penis erfolgen.

Es ist nun vorgeschlagen worden, eine Penisextensionsvorrichtung mit dem Penis mit Hilfe einer hinter der Eichel des Penis angreifenden Schlaufe zu verbinden. Ferner sind Kondome und zylinderförmige Hohlkörper (US 5 707 341 und EP 1 779 822 A1) vorgeschlagen worden.

Aus der DE 20 206 017 667 U1 und DE 20 207 003 824 U1 sind so genannte Eichelschlitten bekannt, auf denen der Penis aufgelegt und mit Hilfe eines Fixierelementes befestigt wird. Das Fixierelement greift hinter der Eichel und somit an der proximalen Seite der Eichel an und verhindert, dass der Penis beim Ausüben eines Zugs von dem Auflageelement weggezogen wird.

Ein Befestigungselement für einen Penis gemäß des Oberbegriffes von Anspruch 1 ist der DE 10200702236 B3 bekannt.

Nachteilig an diesen bekannten Befestigungselementen ist, dass sie entweder den Penis nicht verlässlich fixieren oder aus mehreren Teilen bestehen und umständlich anzulegen sind.

Aufgabe der Erfindung ist es, ein einfach aufgebautes gattungsgemäßes Befestigungselement bereitzustellen, dass einfach aufgebaut ist und eine verlässliche sowie schützende Verbindung zur Penisextensionsvorrichtung gewährleistet.

Gelöst wird diese Aufgabe durch ein erfindungsgemäßes Befestigungselement.

Unter einem Auflageelement wird dabei ein solches Element verstanden, auf das der Penis aufgelegt wird und auf dem der Penis mit Hilfe des Fixierelementes ortsfest lokalisiert wird.

Das erfindungsgemäße Befestigungselement zeichnet sich dadurch aus, dass das Auflageelement mit einem Deckel beweglich verbunden ist. Dieser Deckel kann eine geschlossene Position einnehmen, in der er zusammen mit dem Auflageelement einen rohrförmigen Hohlkörper bildet, in dem der Penis zu liegen kommt. Dieser rohrförmige Hohlkörper besitzt somit ein proximales/körperzugewandtes und ein distales/körperabgewandtes Ende. Der Penis liegt somit im Inneren dieses Hohlkörpers.

Der Deckel kann nicht nur eine geschlossene Position sondern auch eine geöffnete Position einnehmen. Unter einer geöffneten Position wird im Rahmen der vorliegenden Unterlagen nicht eine bestimmte Position bezeichnet. Vielmehr soll dadurch zum Ausdruck gebracht werden, dass der Deckel von der geschlossenen Position in eine andere Position gebracht werden kann, in der Deckel und Auflageelement zusammen kein "geschlossenes Rohr" mehr bilden.

Der rohrförmige Hohlkörper kann im übrigen jeden beliebigen Querschnitt besitzen. Im einfachsten Fall stellt das Auflageelement einen planen Streifen dar, der durch einen rechteckigen oder runden Deckel geschlossen wird, so dass sich insgesamt ein Hohlkörper mit eckenförmigen Querschnitt ergibt. Zweckmäßigerweise ist der Querschnitt des Hohlkörpers jedoch rund oder oval. Mit anderen Worten, der Hohlkörper stellt ein Rohr mit dem entsprechenden Querschnitt dar.

Vorzugsweise bilden das Auflageelement und der Deckel jeweils eine rohrförmige Halbschale. Es ist dabei nicht zwingend, dass beide eine komplette Halbschale und somit die Hälfte eines Rohres bilden. Vielmehr kann beispielsweise das Auflageelement eine Schale bilden, welche nur einen gewissen Bruchteil des sich beim Zusammenfügen von Auflageelement und Deckel ergebenden Vollrohres darstellt. Vorzugsweise sind beide Halbschalen in etwa zylinderrohrförmige in etwa gleich große Halbschalen.

Die Verbindung zwischen Deckel und Auflageelement kann beliebiger Art sein. Es kann sich dabei um Scharniere, Gelenke oder Ähnliches handeln. Vorzugsweise sind die beiden Halbschalen vom Auflageelement und vom Deckel an einer ihrer Längsseite beweglich miteinander verbunden. Mit anderen Worten, zwei im geschlossenen Zustand aneinander anliegende Längsseiten der Halbschale sind beweglich miteinander verbunden.

An den beiden anderen Längsseiten sind beide Halbschalen vorzugsweise mit Verschlussmitteln ausgestattet, die einerseits ein dauerhaftes Verbinden der beiden Halbschalen miteinander und andererseits ein Öffnen dieser Verbindung ermöglichen, so dass Aufnahmeelement und Deckel "aufgeklappt" werden können. Bei diesen Verschlussmitteln kann es sich um übliche handeln. So kann es sich beispielsweise um eine Klipp- oder Einrastverbindung handeln.

Der aus Auflageelement und Deckel gebildete rohrförmige Hohlkörper ist an seinem distalen Ende vorzugsweise verschlossen. An diesem verschlossenem distalen Ende sind das Auflageelement und/oder der Deckel mit mindestens einer Öffnung ausgestattet. Mit anderen Worten, sowohl das Auflageelement als auch der Deckel können am distalen Ende mindestens eine Öffnung besitzen.

Nach einer weiterhin bevorzugten Ausführungsform ist der rohrförmige Hohlkörper an seinem distalen Ende unter Bildung einer Kuppel verschlossen. Diese Kuppel ist vorzugsweise gegenüber dem rohrförmigen Hohlkörper aufgeweitet. In dieser Kuppel kommt zweckmäßigerweise die Eichel zu liegen.

Das Auflageelement und der Deckel sind vorzugsweise aus einem formstabilen Kunststoff gefertigt. Die Elemente, welche eine bewegliche Verbindung von Auflageelement und Deckel bewirken, sind vorzugsweise einstückig damit ausgebildet. Gleiches gilt für die Verschlussmittel.

Das Auflagemittelelement und/oder der Deckel können auch aus einem Gummi oder gummiartigen Material gefertigt sein. Allerdings muss dieses Material über eine solche Härte und/oder Wandstärke verfügen, dass der Hohlkörper auch bei Zugbelastung derartig formstabil ist, dass der sich in dem Hohlkörper befindliche Penis nicht "eingequetscht" wird. Es ist auch möglich, das Auflageelement aus einem formstabilen Kunststoff und den Deckel aus einem wie oben beschriebenen Gummi oder gummiartigen Material anzufertigen. Gleiches gilt umgekehrt. Es muss lediglich sichergestellt werden, dass die Rohrform bzw. die rohrförmige Hohlkörperform auch bei Zuganwendung in etwa erhalten bleibt und der für den Penis zur Verfügung stehende Raum nicht unnötig eingeschränkt bzw. reduziert wird.

Mit dem erfindungsgemäßen Befestigungselement ist es möglich, den Penis verlässlich und dauerhaft mit der dazugehörigen Penisextensionsvorrichtung zu verbinden und einen Zug auszuüben. Ferner wird der Penis geschützt, da er von dem rohrförmigen Hohlkörper vollständig umschlossen ist. Der Penis kann somit im zugbelasteten Zustand nicht an der Unterwäsche oder Ähnlichem reiben, so dass Irritationen vermieden werden. Dies gilt insbesondere für nicht beschnittene Männer, bei denen die Vorhaut zurückgezogen werden muss, damit das Fixierelement auf der proximalen Seite der Eichel angreifen kann, um seine Funktion zu erfüllen.

Um Zug mit Hilfe der Penisextensionsvorrichtung auf das Befestigungselement ausüben zu können, wird vorzugsweise entweder das Auflageelement oder der Deckel an dem distalen Ende mit einem Verbindungselement ausgestattet, welches mit der Extensionsvorrichtung verbunden wird.

Das Fixierelement des erfindungsgemäßen Befestigungselements befindet sich vorzugsweise an dem proximalen Ende oder in der Nähe des proximalen Endes des Befestigungselements.

Die Erfindung wird im folgenden anhand der beiliegenden nicht maßstabsgetreuen und skizzenhaften Zeichnungen näher erläutert. In diesen Zeichnungen zeigen
- Fig. 1: eine perspektivische Ansicht eines erfindungsgemäßen Befestigungselements in einer geöffneten Position,
- Fig. 2: eine perspektivische Ansicht des in der Fig. 1 gezeigten Befestigungselements in der geschlossenen Position,
- Fig. 3: eine Seitenansicht des in der Fig. 2 gezeigten Befestigungselements,
- Fig. 4: eine Querschnittsansicht durch das in der Fig. 3 gezeigte Befestigungselement entlang der Linie X-X,
- Fig. 5: eine Ansicht von vorne auf das in der Fig. 3 gezeigte Befestigungselement und somit eine Ansicht von rechts, bezogen auf die Fig. 3,
- Fig. 6: eine Aufsicht von oben auf das in der Fig. 1 gezeigte Befestigungselement in geöffneter Position mit eingelegtem Penis und
- Fig. 7: eine Vorderansicht einer Person, welche eine Penisextensionsvorrichtung trägt, die mit Hilfe des erfindungsgemäßen Befestigungselementes einen Zug auf den Penis ausübt.

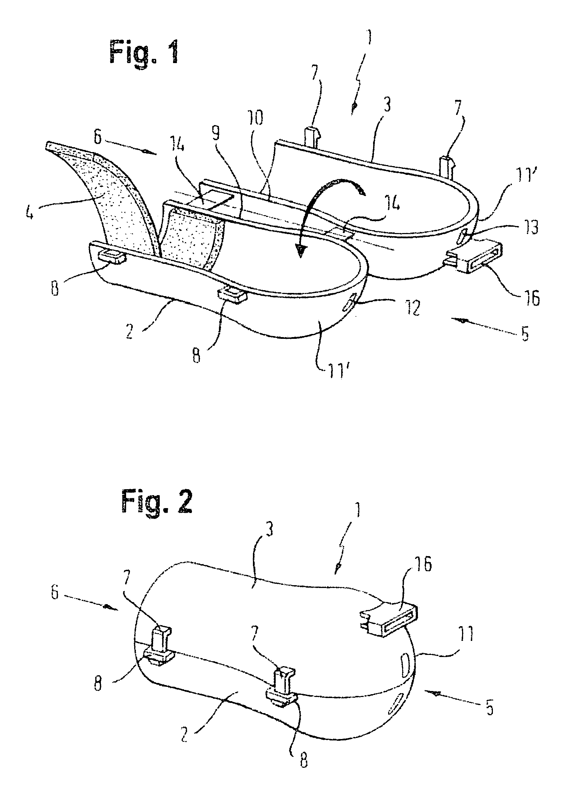

Das in der Fig. 1 in perspektivischer Ansicht gezeigte Befestigungselement 1 besitzt ein Auflageelement 2 und einen Deckel 3, die mit Hilfe von zwei Scharnieren 14 beweglich miteinander verbunden sind. Diese Scharniere 14 greifen in der Nähe oder an den gegenüberliegenden Längsseite 10 vom Deckel 3 und der Längsseite 9 des Auflageelementes 2 an.

Um in die geschlossene, in der Fig. 2 gezeigte Position zu gelangen, wird der Deckel 3 entsprechend dem in der Fig. 1 gezeigten Pfeil geschwenkt, so dass der Deckel 3 auf dem Auflageelement 2 zu liegen kommt und einen rohrförmigen Hohlkörper 2, 3 bildet. Dieser Hohlkörper ist in perspektivischer Ansicht in der Fig. 2 gezeigt und besitzt ein distales Ende 5 sowie ein proximales Ende 6.

Das Auflageelement 2 und der Deckel 3 stellen zwei in etwa gleich große Halbschalen dar, die an dem distalen Ende 5 eine "halbe" Kuppel 11' bilden, die in der in der Figur 2 gezeigten geschlossenen Position zusammen eine "volle" Kuppel 11 bilden.

An diesem distalen Ende 5 ist der rohrförmige Hohlkörper 2, 3 somit durch die Kuppel 11 verschlossen.

In diesem Kuppelbereich ist der rohrförmige Hohlkörper 2,3 gegenüber dem restlichen Bereich des Hohlkörpers 2, 3 aufgeweitet. Mit anderen Worten, der Durchmesser des Hohlkörpers 2, 3 vergrößert sich ausgehend von der proximalen Seite und nimmt dann nach Durchschreiten einem Maximums zum proximalen Ende hin ab, so dass sich die genannte Kuppel 11 ergibt.

Es ist im übrigen nicht zwingend, dass der Hohlkörper 2, 3 am proximalen Ende 5 in der beschriebenen Art und Weise verschlossen ist. Er kann an diesem proximalen Ende 6 auch offen sein. Die Größe der Öffnung kann je nach belieben gewählt werden. Der Hohlkörper 2, 3 kann somit im einfachsten Fall auch aus einem Rohrabschnitt mit gleich bleibendem oder sich auch veränderndem Durchmesser darstellen.

Am proximalen Ende 6ist ein Fixierelement in Form eines Streifens 4 aus einem elastischem Material eingelegt, das zweckmäßigerweise mit dem Auflageelement 2 verbunden ist, beispielsweise durch Verkleben.

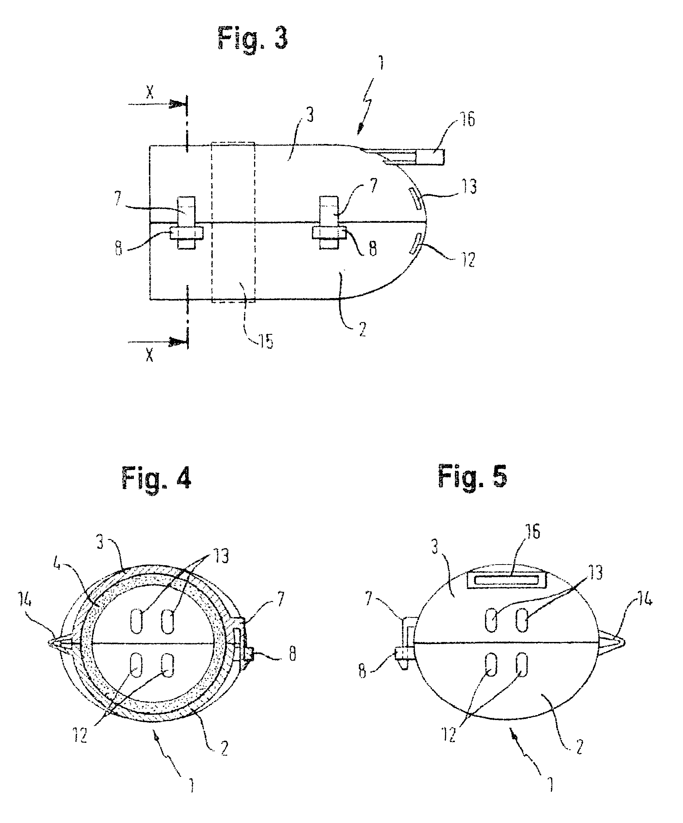

Sowohl der Deckel 3 als auch das Auflageelement 2 besitzen am distalen Ende 5 jeweils zwei Öffnungen 12 bzw. 13, man vergleiche auch Figuren 4 und 5.

Um Deckel 3 und Auflageelement 2 in der geschlossenen Position zu halten, dienen Haken 7 am Deckel 3, die in Ösen 8 am Auflageelement 2 eingreifen und dort einrasten. Diese Haken 7 und Ösen 8 sind auf der den Längsseiten 9 bzw. 10 gegenüberliegenden Längsseiten von Deckel 3 bzw. Auflageelement 2 angeordnet.

Das in den Figuren gezeigte Befestigungselement 1 ist aus einem Kunststoffmaterial gefertigt, beispielsweise spritzgeformt. Bei den Scharnieren 14 kann es sich um Filmscharniere handeln. Diese Filmscharniere 14 und auch die Haken 7 und die Ösen 8 sind einstückig mit dem Deckel 3 bzw. dem Auflageelement 2 spritzgeformt. Zudem handelt es sich bei dem Befestigungselement um ein formstabiles Element, das auch bei Ausüben einer für Penisextensionsgeräte üblichen Kraft bzw. einem üblichen Zug seine Form beibehält oder nur wenig verändert.

An Stelle der Haken 7 und Ösen 8 kann auch ein Klettverschlussband 15 eingesetzt werden, das um das Befestigungselement 1 in der geschlossenen Position herumgewickelt wird. Diesbezüglich wird auf die Figur 3 verwiesen.

Der Deckel 3 ist an seinem distalen Ende 5 mit dem weiblichen Teil 16 eines Blitzverschlusses bzw. einer Klickschnalle ausgestattet, der an den Deckel 3 angeformt ist.

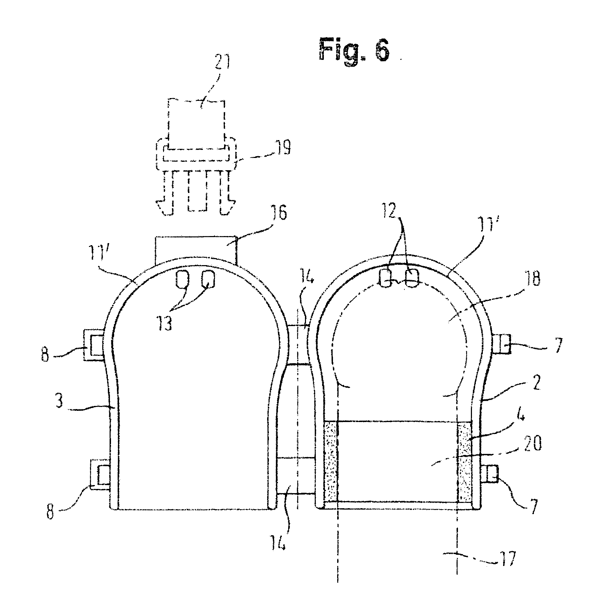

Um das Befestigungselement 1 mit dem Penis 17 zu verbinden wird letzter 17 (man vergleiche Figur 6) in eine horizontale, vom Körper wegzeigende Lage gebracht und auf das Auflageelement 2 aufgelegt bzw. eingelegt. Dabei kommt die Eichel 18 in der Halbkuppel 11' zu liegen.

Wie aus der Figur 6 ersichtlich ist, befindet sich dann das Fixierelement 4 auf der proximalen Seite der Eichel 18.

Anschließend wird der Deckel 3 auf das Auflageelement 2 verschwenkt, so dass der Hohlkörper 2, 3 verschlossen wird. Dabei ist dafür Sorge zu tragen, dass das Fixierelement 4 um den Penisschaft 20 umläuft, um ihn mittig in dem Hohlkörper 2, 3 zu fixieren, so dass der Penis 17 auch nicht bei Anwendung eines Zuges herausgezogen werden kann.

Um diesen Zug an das Befestigungselement 1 anzulegen, wird der Penis 17 zusammen mit dem Befestigungselement 1 zum Körper zurück gegen den Bauch gelegt. In den weiblichen Teil 16 des Blitzverschlusses wird der damit korrespondierende männliche Teil 19 dieses Blitzverschlusses eingeführt. Der männliche Teil 19 ist zudem mit einem Gurt 21 verbunden.

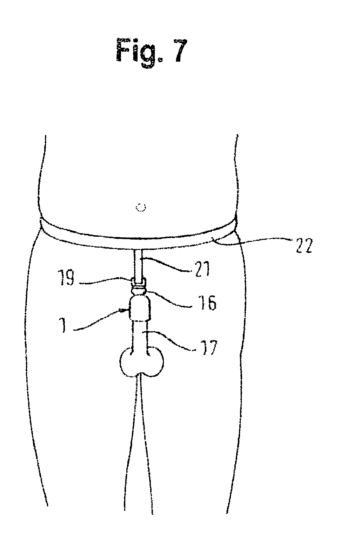

Figur 7 zeigt, wie dieser Gurt 21 mit einem Gürtel 22 oder ähnlichem verbunden ist, welcher um den Bauchbereich einer Person umläuft. Entweder der Gurt 21 oder Gürtel 22 oder auch beide 21, 22 sind aus einem elastischen Material gefertigt. Gurt 21 und/oder Gürtel 22 werden gespannt, so dass über das Befestigungselement 1 eine Zugwirkung auf den Penis 17 ausgeübt wird.

### Bezugszeichenliste

- 1: Befestigungselement
- 2: Auflageelement
- 3: Deckel
- 4: Fixierelement
- 5: distales Ende
- 6: proximales Ende
- 7: Haken
- 8: Öse
- 9: Längsseite Auflageelement 2
- 10: Längsseite Deckel 3
- 11: Kuppel
- 11': Halbkuppel
- 12: Öffnung
- 13: Öffnung
- 14: Scharnier
- 15: Klettverschlussband
- 16: weiblicher Teil Blitzverschluss/Klickschnalle
- 17: Penis
- 18: Eichel
- 19: männlicher Teil Blitzverschluss/Klickschnalle
- 20: Penisschaft
- 21: Gurt
- 22: Gürtel

## Patentansprüche

1. Befestigungselement (1) für einen Penis (17) zum Verbinden mit einer Penisextensionsvorrichtung, das mit einem Auflageelement (2) sowie einem Fixierelement (4) für den Penis (17) ausgestattet ist,
**dadurch gekennzeichnet, dass**
das Auflageelement (2) mit einem Deckel (3) beweglich verbunden ist,
der Deckel (3) eine geöffnete und eine geschlossene Position einnehmen kann und
das Auflageelement (2) zusammen mit dem Deckel (3) in der geschlossenen Position einen rohrförmigen Hohlkörper (2, 3) mit einem proximalen (6) und einem distalen (5) Ende bilden.

2. Befestigungselement nach Anspruch 1,
**dadurch gekennzeichnet, dass**
das Auflageelement (2) und der Deckel (3) jeweils eine rohrförmige Halbschale darstellen und die beiden Halbschalen in der geschlossenen Position ein den Hohlkörper (2, 3) bildendes Vollrohr ergeben.

3. Befestigungselement nach Anspruch 1 oder 2,
**dadurch gekennzeichnet, dass**
beide Halbschalen in etwa zylinderrohrförmige Halbschalen sind.

4. Befestigungselement nach Anspruch 3,
**dadurch gekennzeichnet, dass**
beide Halbschalen an einer ihren Längsseiten (9 bzw. 10) beweglich miteinander verbunden sind.

5. Befestigungselement nach Anspruch 4,
**dadurch gekennzeichnet, dass**
beide Halbschalen an ihrer anderen Längsseite mit Verschlussmitteln (7, 8) ausgestattet sind, die einerseits ein dauerhaftes Verbinden der beiden Halbschalen miteinander und andererseits ein Öffnen dieser Verbindung ermöglichen.

6. Befestigungselement nach einem der vorhergehenden Ansprüche,
**dadurch gekennzeichnet, dass**
der rohrförmige Hohlkörper (2, 3) an seinem distalen Ende (5) verschlossen ist.

7. Befestigungselement nach Anspruch 6,
**dadurch gekennzeichnet, dass**
das Auflageelement (2) und/oder der Deckel (3) am distalen Ende (5) jeweils mit mindestens einer Öffnung (12 bzw. 13) ausgestattet ist/sind.

8. Befestigungselement nach Anspruch 6 oder 7,
**dadurch gekennzeichnet, dass**
der rohrförmige Hohlkörper (2, 3) am distalen Ende (5) unter Bildung einer Kuppel (11) verschlossen ist.

9. Befestigungselement nach Anspruch 8,
**dadurch gekennzeichnet, dass**
die Kuppel (11) gegenüber dem rohrförmige Hohlkörper (2, 3) aufgeweitet ist.

10. Befestigungselement nach einem der vorhergehenden Ansprüche,
**dadurch gekennzeichnet, dass**
das Auflageelement (2) und der Deckel (3) aus einem formstabilen Kunststoff oder einem flexiblen Material gefertigt sind.

11. Befestigungselement nach einem der vorhergehenden Ansprüche,
**dadurch gekennzeichnet, dass**
entweder das Auflageelement (2) oder der Deckel (3) an dem distalen Ende (5) ein Verbindungselement (16) mit der Penisextensionsvorrichtung aufweist.

12. Befestigungselement nach einem der vorhergehenden Ansprüche,
**dadurch gekennzeichnet, dass**
das Fixierelement (4) an dem proximalen Ende (6) oder in der Nähe des proximalen Endes (6) angeordnet ist.

## Claims

1. A fastening element (1) for a penis (17) for connecting to a penis extension device provided with a supporting element (2) as well as a fixing element (4) for the penis (17),
**characterized in that**
the supporting element (2) is connected to the cover (3) so as to be movable, the cover (3) can assume an open and a closed position, and
when in the closed position, the supporting element (2) together with the cover (3) forms a tubular hollow body (2, 3) having a proximal end (6) and a distal end (5).

2. The fastening element according to claim 1,
**characterized in that**
the supporting element (2) and the cover (3) each form a tubular half shell, and, when in the closed position, the two half shells yield a full tube forming the hollow body (2, 3).

3. The fastening element according to claim 1 or 2,
**characterized in that**
the two half shells are approximately cylindrical half shells.

4. The fastening element according to claim 3,
**characterized in that**
the two half shells are movably connected to one another on one of their longitudinal sides (9 or 10).

5. The fastening element according to claim 4,
**characterized in that**
the two half shells are provided with closing means (7, 8) on their other longitudinal side thereof which, on the one hand, enable the two half shells to be permanently connected to each other and, on the other, the connection to be released.

6. The fastening element according to any one of the preceding claims,
**characterized in that**
the tubular hollow body (2, 3) is closed at its distal end (5).

7. The fastening element according to claim 6,
**characterized in that**
the supporting element (2) and/or the cover (3) is/are provided with at least one respective opening (12 or 13) at the distal end (5).

8. The fastening element according to claim 6 or 7,
**characterized in that**
the tubular hollow body (2, 3) is shut at the distal end (5) while forming a dome (11).

9. The fastening element according to claim 8,
**characterized in that**
the dome (11) is wider than the tubular hollow body (2, 3).

10. The fastening element according to any one of the preceding claims,
**characterized in that**
the supporting element (2) and the cover (3) are manufactured from a dimensionally stable plastic material or a flexible material.

11. The fastening element according to any one of the preceding claims,
**characterized in that**
either the supporting element (2) or the cover (3) has a connecting element (16) to the penis extension device at the distal end (5).

12. The fastening element according to any one of the preceding claims,
**characterized in that**
the fixing element (4) is arranged at the proximal end (6) or close to the proximal end (6).

## Revendications

1. Elément de fixation (1) pour un pénis (17) pour le raccordement à un dispositif d'extension de pénis, l'élément étant équipé d'un élément d'appui (2) ainsi que d'un élément d'attachement (4) pour le pénis (17),
**caractérisé en ce que**
l'élément d'appui (2) est raccordé de façon mobile à un couvercle (3),
le couvercle (3) peut adopter une position ouverte et une position fermée et
l'élément d'appui (2) forme avec le couvercle (3), dans la position fermée, un corps creux (2, 3) tubulaire ayant une extrémité proximale (6) et une extrémité distale (5).

2. Elément de fixation selon la revendication 1, **caractérisé en ce que** l'élément d'appui (2) et le couvercle (3) représentent respectivement une demi-coque tubulaire, et **en ce que** les deux demi-coques fournissent, dans la position fermée, un tube complet formant le corps creux (2, 3).

3. Elément de fixation selon la revendication 1 ou 2, **caractérisé en ce que** les deux demi-coques sont des demi-coques approximativement en forme de tube cylindrique.

4. Elément de fixation selon la revendication 3, **caractérisé en ce que** les deux demi-coques sont, sur un de leurs grands côtés (9 ou respectivement 10), raccordées l'une à l'autre de façon mobile.

5. Elément de fixation selon la revendication 4, **caractérisé en ce que** les deux demi-coques sont, sur leur autre grand côté, équipées de moyens de fermeture (7, 8) qui permettent d'une part un raccordement durable des deux demi-coques et permettent d'autre part d'ouvrir ce raccordement.

6. Elément de fixation selon l'une des revendications précédentes, **caractérisé en ce que** le corps creux (2, 3) tubulaire est fermé à son extrémité distale (5).

7. Elément de fixation selon la revendication 6, **caractérisé en ce que** l'élément d'appui (2) et/ou le couvercle (3) est/sont équipé(s) à l'extrémité distale (5) respectivement d'au moins une ouverture (12 ou respectivement 13).

8. Elément de fixation selon la revendication 6 ou 7, **caractérisé en ce que** le corps creux (2, 3) tubulaire est, à l'extrémité distale (5), fermé avec formation d'une coupole (11).

9. Elément de fixation selon la revendication 8, **caractérisé en ce que** la coupole (11) est évasée par rapport au corps creux (2, 3) tubulaire.

10. Elément de fixation selon l'une des revendications précédentes, **caractérisé en ce que** l'élément d'appui (2) et le couvercle (3) sont réalisés en matière plastique indéformable ou en matériau flexible.

11. Elément de fixation selon l'une des revendications précédentes, **caractérisé en ce que** soit l'élément d'appui (2) soit le couvercle (3) présente, à l'extrémité distale (5), un élément de raccordement (16) au dispositif d'extension de pénis.

12. Elément de fixation selon l'une des revendications précédentes, **caractérisé en ce que** l'élément d'attachement (4) est disposé sur l'extrémité proximale (6) ou à proximité de l'extrémité proximale (6).
